(19) Europäisches Patentamt
European Patent Office
Office européen des brevets

(11) Publication number: **0 146 090 B1**

(12) **EUROPEAN PATENT SPECIFICATION**

(45) Date of publication of patent specification: **18.03.92** (51) Int. Cl.5: **C07H 3/06**, A61K 31/70

(21) Application number: **84114925.5**

(22) Date of filing: **07.12.84**

(54) **Sialyloligosaccharides and method for producing the same.**

(30) Priority: **08.12.83 JP 232043/83**

(43) Date of publication of application:
**26.06.85 Bulletin 85/26**

(45) Publication of the grant of the patent:
**18.03.92 Bulletin 92/12**

(84) Designated Contracting States:
**CH DE FR LI SE**

(56) References cited:

**CARBOHYDRATE RESEARCH, vol. 127, April 1984, pages C1-C4; Elsevier Science Publ. B.V., Amsterdam, NL T. KITAJIMA et al.: "Synthesis of a linear tetrasaccharide unit of a complex type of glycan chain of a glycoprotein."**

**ANGEWANDTE CHEMIE, vol. 21, no. 12, 1982, pages 927-928; Verlag Chemie GmbH, Weinheim, DE H. PAULSEN et al.: "Synthesis of trisaccharide moieties form N-acetylneuraminic acid and N-acetyllactosamine."**

(73) Proprietor: **RIKAGAKU KENKYUSHO**
**2-1 Hirosawa**
**Wako-shi Saitama-ken(JP)**

Proprietor: **Towa Chemical Industry Co.., Ltd**
**1-2, Ohtemachi 2-chome**
**Chiyoda-ku Tokyo(JP)**

Proprietor: **MECT CORPORATION**
**1-1, Nishishinjuku 2-Chome**
**Shinjuku-ku Tokyo 163(JP)**

(72) Inventor: **Ogawa, Tomoya**
**No. 3-6-6-3-101, Kichijyouji Kita-machi**
**Musashino-shi Tokyo(JP)**
Inventor: **Nukada, Tomoo**
**No. 5-19-9, Nishiogi Kita**
**Suginami-ku Tokyo(JP)**
Inventor: **Kitajima, Toru**
**No. 4-31-2, Nagasaki**
**Toshima-ku Tokyo(JP)**
Inventor: **Sugimoto, Mamoru**
**No. 4-6-32, Sakae**
**Niiza-shi Saitama-ken(JP)**
Inventor: **Takemura, Motohiro**
**No. 615-8, Shimoishido Shimo**
**Kitamoto-shi Saitama-ken(JP)**

**EP 0 146 090 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

JOURNAL OF THE CHEMICAL SOCIETY, PERKIN TRANSACTIONS I, 1981, pages 2070-2074, The Royal Soc. of Chemistry, London, GB, J. ARNARP et al.: "Synthesis of a tri-, a penta-, and a hepta-saccharide containin terminal N-acetyl-beta-D-lactosaminyl residues, part of the 'complex-type' carbohydrate moiety of glycoproteins."

Carbohydrate Research, 123(1983)C8-C11 (publication November 1983)

Inventor: **Shitori, Yoshiyasu**
**No. 3-5-24-408, Naka-cho**
**Musashino-shi Tokyo(JP)**

(74) Representative: **Vossius & Partner**
**Siebertstrasse 4 P.O. Box 86 07 67**
**W-8000 München 86(DE)**

## Description

This invention relates to new sialyloligosaccharides which correspond to the non-reducing terminal tetrasaccharide unit of N-glycosidic sugar chain of glycoprotein such as borine blood coagulation factor II (see E.G. Berger et all Experientia 38 (1982) 1129 - 1133), and to the method for producing the same.

NeuAcα 2→ 6Galβ 1→ 4GlcNAcβ 1→ 2Manα 1 → 6Manβ 1→4GlcNAcβ 1→
NeuAcα 2→ 6Galβ 1→ 4GlcNAcβ 1→ 2Manα 1 → 3

4GlcNAcβ1 → Asn

| | |
|---|---|
| NeuAc : | N-acetyl neuraminic acid |
| Gal : | Galactose |
| Glc : | Glucose |
| Man : | Mannose |
| GlcNAc : | N-acetyl glucosamine |
| Asn : | Asparagine |

Sialic acid exists in a free or a combined form with sialoylglycoconjugate such as glycoprotein, glycolipid, oligosaccharide, polysaccharide, etc. in the surface of animal or bacterial cells and much attention is now focused thereon from a medical and pharmacological point of view as a compound showing effectivity in the fields of immunity, cancer, inflammation, virosis, cytodieresis, hormone reception, etc.

With increase in use of chemotherapeutics such as adrenocortical hormones or immunosuppressant for the treatment of various diseases such as carcinosis, hypoimmunity and many kinds of side effects are observed and therefore, it is becoming more difficult to treat a patient suffering from such diseases as cancer.

J. Chem. Soc., Perkin Trans. I 1981, 2070 relates to the synthesis of a tri-, a penta-, and a hepta-saccharide containing terminal N-acetyl-β-D-lactosaminyl residues, part of the "complex-type" carbohydrate moiety of glycoproteins.

Furthermore the reference Carbohydrate Research 123(1983)C8-C11 relates to the synthesis of a nonahexosyl unit which is part of a glycon chain of a glycoprotein starting from a trihexosyl donor.

The inventors have paid attention to the importance of sialic acid, which is one of the inherent constituents of animal living body, as a marker on the cell surface and have conducted reactions of sialic acid halide as a glycosyl donor with various glycosyl acceptors. As a result, they have succeeded in the synthesis of new sialyloligosaccharides which are expected to cause no side effects and to possess many desired physiological activities such as adaptation of immunoresponse and have accomplished this invention.

This invention provides sialyloligosaccharides of the general formula described below and a method for producing the same:

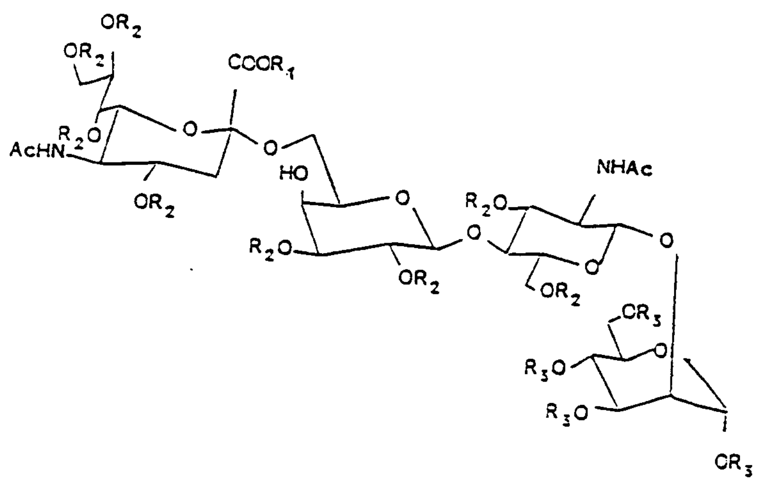

or

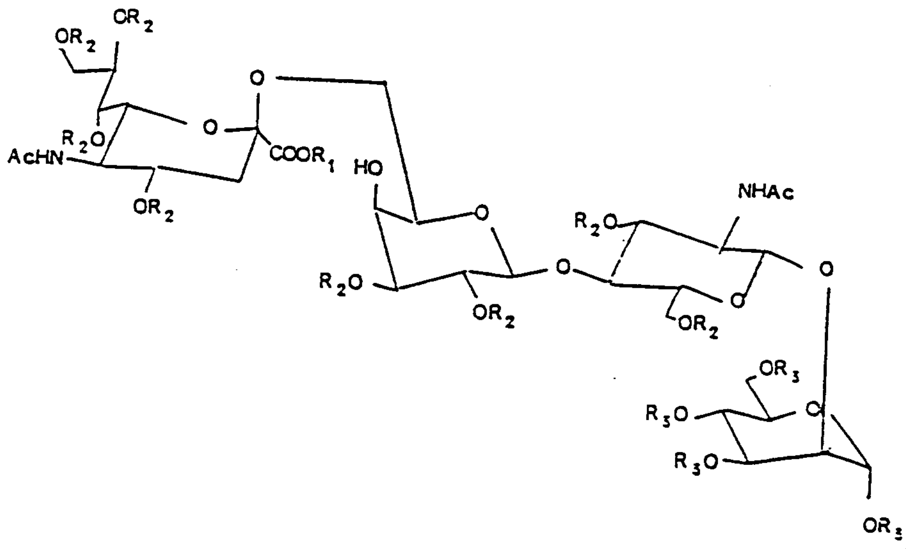

wherein $R_1$ is hydrogen or methyl, $R_2$ is hydrogen or acetyl and $R_3$ is hydrogen or benzyl, and Ac is acetyl.

Furthermore the present invention provides sialyloligosaccharides according to anyone of claims 2 to 7, a process of preparation according to claims 8, intermediate compounds according to anyone of claims 9 to 11, 13 and 14 and a process of preparation of intermediate compounds according to claim 12. Additionally the present invention comprises pharmaceutical preparations according to claim 15.

The sialyloligosaccharides of the above general formula according to the present invention can be prepared by reacting the glycosyl donor of the general formula (12), which can be derived from sialic acid by a conventional method, with the glycosyl acceptor, trisaccharide of the formula (10) to form tetrasaccharide and optionally removing acetyl, methyl or benzyl groups:

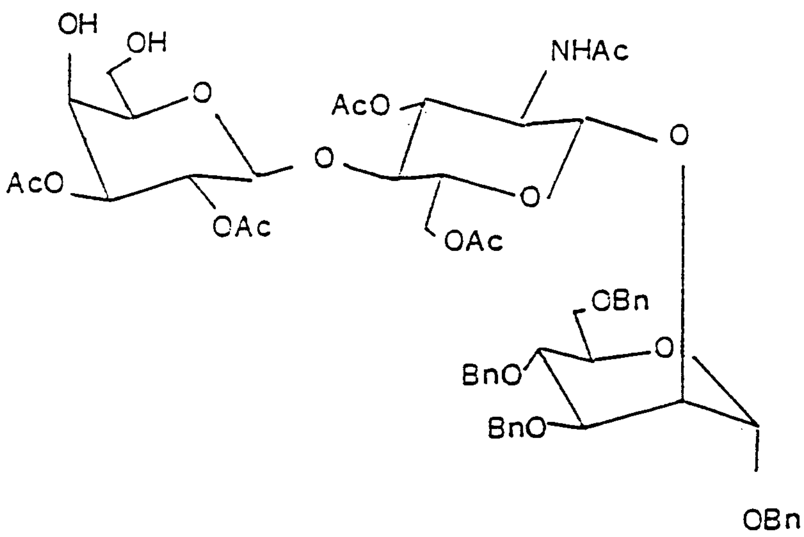

(10)

wherein Ac represents acetyl and Bn represents benzyl,

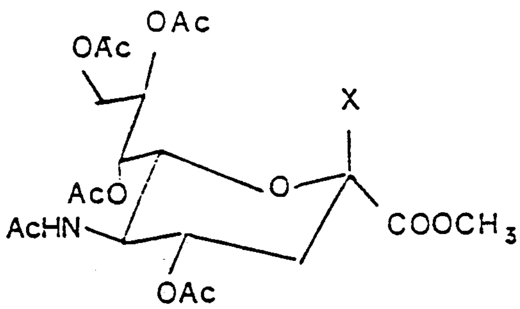

(12)

wherein X represents halogen, preferably Cl or Br.

The glycosyl donor (12), the starting compound of the present invention, can be derived from sialic acid methyl ester peracetate (11) by Kuhn et al. method (Chem. Ber., 99 611-617 (1966)).

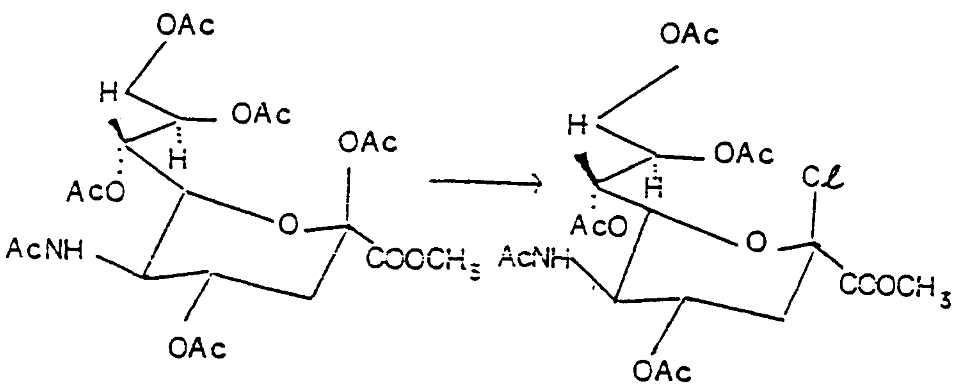

(11)

The glycosyl acceptor (10), the other starting materials of the present invention, can be derived from the compound (6) which can be prepared as follows:

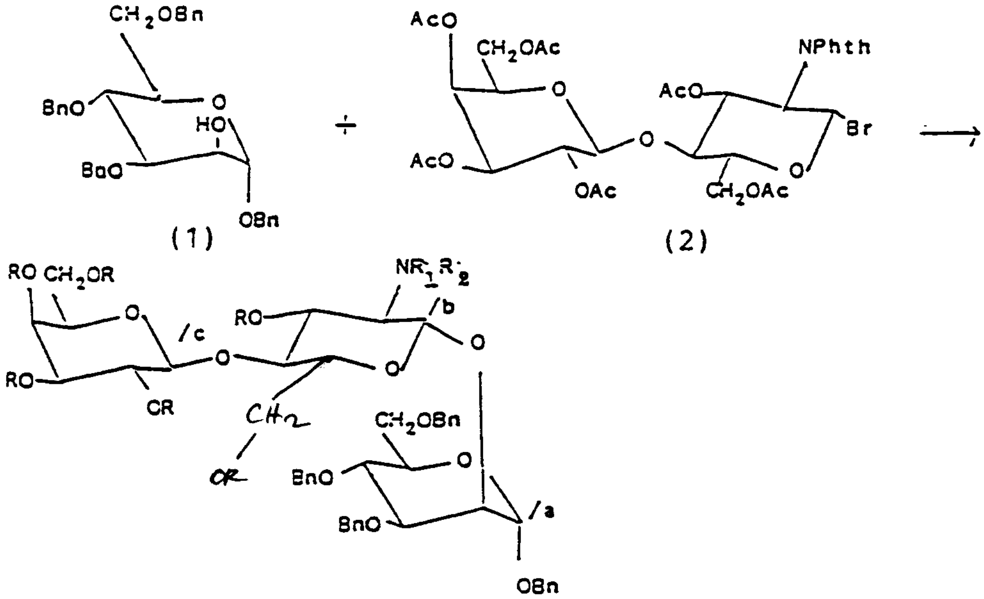

(1)

(2)

(3) R = Ac, $R_1,R_2$ = Phth

(4) R = H, $R_1,R_2$ = Phth

(5) R = H, $R_1,R_2$ = $H_2$

(6) R = Ac, $R_1,R_2$ = H,Ac

(Phth represents phthaloyl)

The compound (1) is reacted with the disaccharide donor (2) in the presence of $AgOSO_2CF_3$ and molecular sieves 4A powder to give the trisaccharide (3) which is treated with $NaOCH_3/CH_3OH$ to give the compound (4) which is then treated with $CH_3OH$-butylamine to give the compound (5) which is subsequently treated with $Ac_2O$-pyridine to give the compound (6).

The compound (1) can be prepared by, for example, the method described in T. Ogawa, H. Yamamoto, Carbohydr. Res., 104 (1982) 271-283. The compound (2) can be prepared by the method described in M.M Ponpipom, R.L. Buglanesi, T.Y. Shen, Tetrahedron. Lett., (1978) 1717-1720; J. Arnarp, J. Lonngren, J. Chem. Commun., (1980) 1000-1002; J. Chem. Soc. Perkin Trans. 1, (1981) 2070-2074; T. Ogawa, S. Nakabayashi, Carbohydr. Res., 97 (1981) 81-86; P.U. Lemieux. S.Z. Abbas, B.Y. Chung, Can. J. Chem., 60 (1982) 58-62.

The glycosyl acceptor (10) can be derived from the resulting compound (6) as shown below.

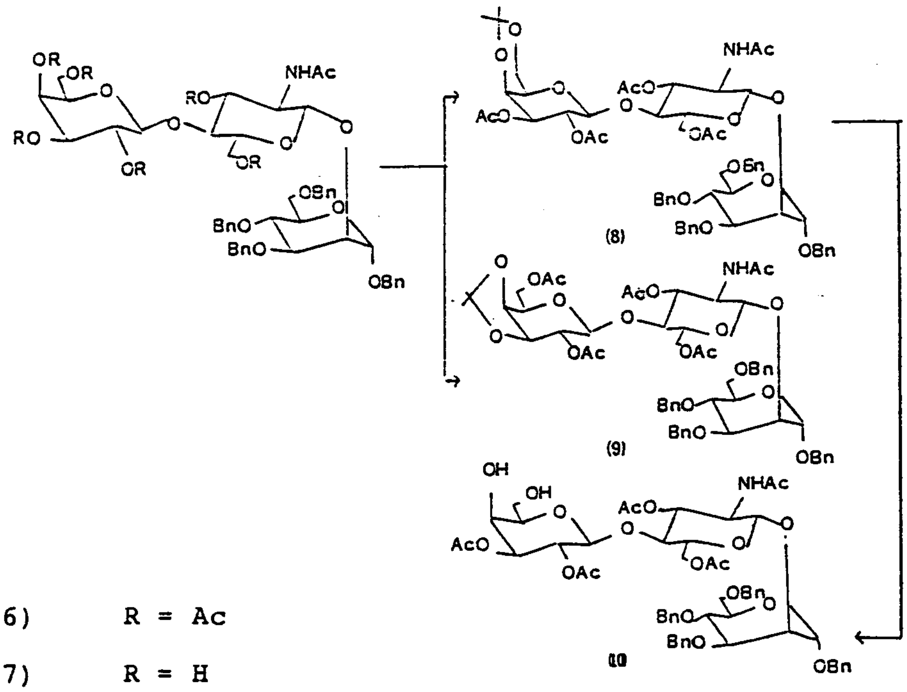

(6) R = Ac

(7) R = H

(8)

(9)

(10)

The compound (6) is deacetylated by alkali metal alkoxide/alcohol, such as $NaOCH_3/CH_3OH$ to obtain the compound (7) which is then reacted with $(CH_3)_2C(OCH_3)_2$ in the presence of catalyst such as p-toluenesulfonic acid (p-TsOH) and subsequently acetylated by, for example, acetic anhydride/pyridine to give 4,6-O-isopropylidene derivative (8) and 3,4-O-isopropylidene derivative (9). The compound (8) is treated with acetic acid/methanol to obtain the glycosyl acceptor (10).

The glycosyl acceptor (10) is reacted with the glycosyl donor (12) in such solvent as dichloromethane, 1,2-dichloroethane, etc. in the presence of glycosidation catalyst such as $Hg(CN)_2$, $HgBr_2$, molecular sieves, $Ag_2CO_3$, $AgClO_4$, $AgOSO_2$ $CF_3$, $(CH_3)_3COSO_2Cr_3$, etc. at -20°C to 150°C, preferably -5°C to 20°C, for 1 to 120 hours, preferably 1 to 5 hours to give $\alpha$-anomer (13) and $\beta$-anomer (14) which can optionally be deacetylated, saponified and debenzylated to obtain $\alpha$-anomer (15) and $\beta$-anomer (16), respectively. Deacetylation, saponification and debenzylation can be performed in conventional systems such as $NaOCH_3/CH_3OH$, NaOH/tetrahydrofuran, or palladium on charcoal/hydrogen system, respectively.

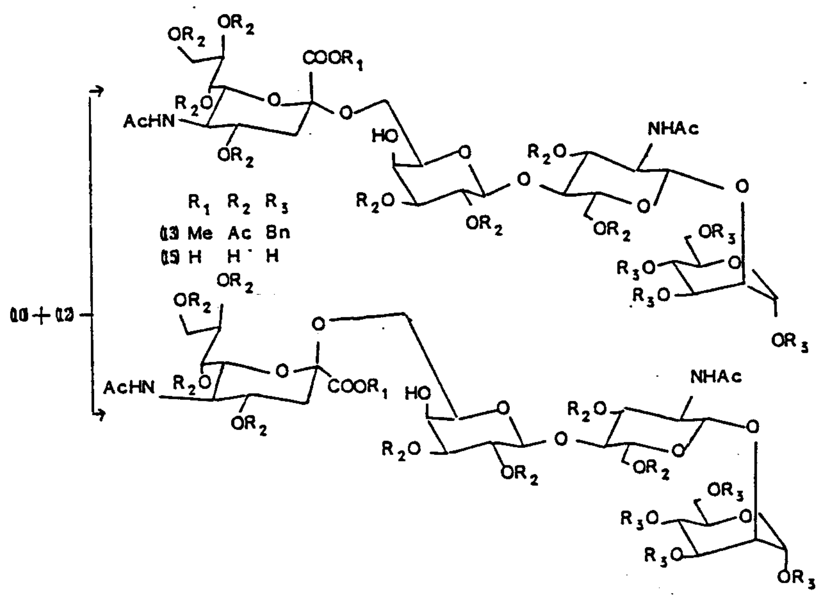

| | $R_1$ | $R_2$ | $R_3$ |
|---|---|---|---|
| (14) | Me | Ac | Bn |
| (16) | H | H | H |

All the compounds (7), (8), (9), (10), (13), (14), (15) and (16) thus obtained are new and have never been disclosed in any references.

These compounds of the present invention may be employed as useful intermediates for the synthesis of the aforementioned sialoylglycoconjugate. They are furthermore expected to show activity as recognition markers on the cell surface and as antagonists.

The present invention will now be explained in detail with reference to the following examples to which the present invention is not limited.

Example 1

Molecular sieves 4A powder (10 g) was vacuum-dried at 190°C for 21 hours, to which $AgOSO_2CF_3$ (3.4 g, 13.2 mmol) and 1,2-dichloroethane (40 ml) were added and argon was substituted for air in the reaction system. Compound (1) (1.6 g, 3.0 mmol) dissolved in 1,2-dichloroethane (20 ml) was added and the reaction mixture was cooled to -20°C. Compound (2) (3.5 g, 4.4 mmol) dissolved in 1,2-dichloroethane (50 ml) was added dropwise. The reaction was performed for 17 hours at -20°C to room temperature. After the reaction was completed, methylene dichloride (500 ml) was added. The reaction mixture was filtered through Celite (registered trademark), washed with water and sodium bicarbonate solution, and dried on $MgSO_4$. After filtration, the filtrate was concentrated in vacuo. The residue was subjected to column chromatography (silica gel 300 g, toluene/ethyl acetate = 3/1) to give the compound (3) (3.45 g, 93.2% from the compound (1)) as oily syrup.

[The compound (3)]

TLC Rf = 0.39 (toluene/ethyl acetate = 2/1)

| Analysis: Calcd. for $C_{66}H_{71}NO_{23}$ • 3/4$C_6H_5CH_3$: | | | |
|---|---|---|---|
| | C, 65.06; | H,5.90; | N,1.06 |
| Found | C,65.15; | H,5.95; | N,1.06 |

$$[\alpha]_D^{23.5} = +4.2^{\circ} \ (C = 1.03,\ CHCl_3)$$

Example 2

Compound (3) (2.2 g) was dissolved in methanol (40 ml) and 1N-$NaOCH_3$ (1 ml) was added thereto. The reaction mixture was stirred at room temperature for 22 hours and concentrated in vacuo. The residue was dissolved in methanol (100 ml), to which n-butylamine (20 ml) was added and refluxed in a bath at 100°C for 45 hours. The reaction mixture was concentrated in vacuo. The residue was dissolved in pyridine (30 ml), to which acetic anhydride (30 ml) was added and stirred at room temperature for 18 hours. The reaction mixture was concentrated in vacuo. The residue was subjected to column chromatography (silica gel 200 g, toluene/ethyl acetate = 1/1) to give the compound (6) (1.9 g, 95.0%) as syrup.

[The compound (6)]

TLC : Rf = 0.54 ($CH_2Cl_2$/acetone = 5/1)

| Analysis: Calcd. for $C_{60}H_{71}NO_{22}$: | | | |
|---|---|---|---|
| | C, 62.22; | H, 6.18; | N, 1.21 |
| Found | C, 62.57; | H, 6.31; | N, 1.50 |

$[\alpha]_D^{21}$ + 6.5° (C = 1.15, $CHCl_3$)

Example 3 Synthesis of the compound (7)

The compound (6) (1.9 g) was dissolved in methanol (40 ml), to which 1N-$NaOCH_3$ (1 ml) was added and stirred at room temperature for 19 hours. AMBERLITE (registered trademark) CG-50 TEE 2 (1 ml) was added to the reaction mixture and stirred for 10 minutes to neutralize it. The reaction mixture was filtered through Celite®, concentrated in vacuo to give quantitatively the compound (7) (1.5 g) as crystals.

[The compound (7)]

TLC: Rf = 0.48 ($CHCL_3$/$CH_3OH$ = 3/1)

| Analysis: Calcd for $C_{48}H_{59}NO_{16}$.1/2 $H_2O$ | | | |
|---|---|---|---|
| | C, 63.01; | H, 6.61; | N, 1.53 |
| Found | C, 63.03; | H, 6.73; | N, 1.77 |

PMR: $^1$H-NMR($CD_3OD$): 7.30 (20H, s, aromatic)
1.93 (3H, s, $COCH_3$)

Example 4 Synthesis of the compounds (8) and (9)

The compound (7) (304.9 mg) was dissolved in dimethylformamide (3 ml), to which $(CH_3)_2C(OCH_3)_2$ - (0.2 ml, 5 eq.) and p-toluene sulfonic acid (10 mg) was added and stirred at room temperature for 15 hours. Triethylamine (1 ml) was added to the reaction mixture which was then concentrated in vacuo. The residue was dissolved in pyridine (5 ml), to which acetic anhydride (5 ml) was added and stirred at room temperature for 18 hours. The reaction mixture was concentrated in vacuo. The residue was subjected to column chromatography (silica gel 30 g, toluene/ethyl acetate = 2/3 containing 1% triethylamine) to give the compound (8) (256.0 mg, 68.3%) as syrup and the compound (9) (39.4 mg, 10.5%) as syrup.

[The compound (8)]

TLC: Rf: 0.40 (toluene/ethyl acetate = 1/3)

(The compound (a), Rf = 0.53)

| Analysis: Calcd. for $C_{59}H_{71}NO_{20}$ | | | |
|---|---|---|---|
| | C: 63.60, | H: 6.42, | N: 1.26 |
| Found | C: 63.36, | H: 6.48, | N: 1.33 |

$[\alpha]_D^{23.5}$ + 23.5° (C = 1.33, $CHCl_3$)

PMR ($CDCl_3$) 7.4 - 7.1 (20H, m, aromatic) 2.12 (3H, s, Ac), 2.04 (6H, s, Ac x 2) 1.97 (3H, s, Ac), 1.80 (3H, s, Ac), 1.42 and 1.36 (2 x 3H, s, isopropylidene)

CMR ($CDCl_3$) 100.84 (C-1c, $^1J_{CH}$159.7Hz), 99.08 (C-1b, $^1J_{CH}$158.7Hz),

98.94 ( 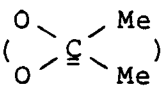 ),

96.74 (C-1a, $^1J_{CH}$167.2Hz),

Example 5 Synthesis of the compound (10)

The compound (8) (602.4 mg) was dissolved in 50% acetic acid/$CH_3OH$ (30 ml) and stirred in a bath at 80°C for 2 hours. The reaction mixture was concentrated in vacuo. The residue was subjected to column chromatography (silica gel 60 g, $CHCl_3$/$CH_3OH$ = 15/1) to give the compound (10) (466.7 mg, 80.4%) as syrup.

[The compound (10)]

TLC: Rf = 0.54 ($CHCl_3$/$CH_3OH$ = 10/1)

| Analysis: Calcd. for $C_{56}H_{67}NO_{20}.H_2O$ | | | |
|---|---|---|---|
| | C, 61.58; | H, 6.37; | N, 1.28 |
| Found | C, 61.56; | H, 6.24; | N, 1.30 |

$[\alpha]_D^{23.5}$ + 12.5° (C1.18, $CHCl_3$)

PMR ($CDCl_3$) : 7.5 - 7.1 (20 H, m, aromatic) 2.08 (3H, s, Ac), 2.04 (6H, s, Ac x 2), 1.98 (3H, s, Ac) 1.80 (3H, s, Ac)

CMR ($CDCl_3$): 101.76 (C-1c, $^1J_{CH}$159.9Hz) 97.62 (C-1b, $^1J_{CH}$159.9Hz) 96.50 (C-1a, $^1J_{CH}$167.2Hz)

Example 6 Synthesis of the compounds (13) and (14)

A molecular sieve 4A powder (0.5 g) was vacuum-dried at 190°C for 20 hours, to which Hg $(CN)_2$ (101.1 mg, 0.40 mmol), $HgBr_2$ (144.2 mg, 0.40 mmol) and 1,2-dichloroethane (2 ml) were added. Argon was substituted for air in the reaction system, to which the glycosyl acceptor (10) (111.3 mg, 0.10 mmol) dissolved in 1,2-dichloroethane (1 ml) was added and stirred at room temperature. The glycosyl donor (12) (101.9 mg, 0.20 mmol) dissolved in 1,2-dichloroethane (1 ml) was dropwise added to the reaction mixture and stirred at room temperature for 43 hours. The glycosyl donor (12) (101.9 mg, 0.20 mmol) dissolved in 1,2-dichloroethane (1 ml) was further added to the reaction mixture and stirred for 68 hours. After the reaction was completed, 50 ml of $CH_2Cl_2$ was added to the reaction mixture which was then filtered through

Celite®, washed with water and sodium bicarbonate solution, and dried over $MgSO_4$. After filtration, the reaction mixture was concentrated in vacuo. The residue was subjected to column chromatography (silica gel 30 g, ethyl acetate) to separate the compounds derived from the compound (12), which were further subjected to column chromatography (silica gel 30 g, $CHCl_3/CH_3OH$ = 40/1) to give α-anomer (13) (54.2 mg, 33.8%) and β-anomer (14) (46.6 mg, 29.1%), as syrup, respectively.

[The compound (13) (α-anomer)]

TLC: Rf = 0.19 ($CHCl_3$/MeOH = 20/1)

| Analysis: Calcd. for $C_{76}H_{94}N_2O_{32}\cdot 2H_2O$ | C, 57.64; | H, 6.24; | N, 1.77 |
|---|---|---|---|
| Found | C, 57.52; | H, 5.96; | N, 1.75 |

$[\alpha]_D^{29}$ + 0.5° (C0.94, $CHCl_3$)
PMR(400 MHz) ($CDCl_3$) : 10 x Ac, s (1,828, 1,886, 1,986, 2,034, 2,041, 2,048, 2,067, 2,071, 2,128): 3,821, (OMe, 3H, s); 2,591 (H-3d, eq, 1H, q, j, 4.39, 12.6)

[The compound (14) (β-anomer)]

TCL: Rf = 0.24 ($CHCl_3$/MeOH = 20/1)

| Analysis: Calcd. for $C_{76}H_{94}N_2O_{32}.3/2H_2O$ | C, 57.97; | H, 6.21; | N, 1.78 |
|---|---|---|---|
| Found | C, 58.00; | H, 6.04; | N, 1.79 |

$[\alpha]_D^{29}$ + 4.0° (C0.50, $CHCl_3$)
PMR (400 MHz) ($CDCl_3$): 10 x Ac, s (1,789, 1,892, 1,998, 2,006, 2,023, 2,039, 2,051, 2,078, 2,120, 2,154; 3,830 (3H, s, OMe) , 2,464 (1H, q, J, 4.88, 12.94 Hz)

Example 7 Synthesis of the compound (15)

The compound (13) (17.7 mg) was dissolved in methanol (2 ml) and 1N - $NaOCH_3$ (0.1 ml) was added thereto. The reaction mixture was stirred at room temperature for 20 hours and concentrated in vacuo. The residue was dissolved in methanol (1 ml), and tetrahydrofuran (1 ml) and $H_2O$ (one drop) were added thereto. The reaction mixture was stirred at room temperature for 19 hours. AMBERLYST (registered trademark) 15 (0.4 ml) was added and stirred for 10 minutes to neutralize the reaction mixture which was then filtered through Celite and concentrated in vacuo. The residue was dissolved in ethanol (2.7 ml), to which 10% palladium on charcoal (20 mg) suspended in water (0.3 ml) was added and hydrogen gas was introduced. The reaction mixture was stirred at room temperature for 18 hours and further at 60°C for 4 hours. After Celite filtration, the reaction mixture was concentrated in vacuo. The residue was subjected to gel permeation chromatography (SEPHADEX G-25 (registered trademark), 20 ml, $H_2O$). The eluate was freeze-dried to give the compound (15) (7.6 mg, 79.2%) as crystals.

[The compound (15)]

TLC: Rf = 0.20 (n-BuOH: $C_2H_5OH$: $H_2O$ = 2:1:1)

$$[\alpha]_D^{26.5} - 20.0^\circ \ (C0.30,\ H_2O)$$

PMR (400 MHz) ($D_2O$) 5,223 (H - 1a, d, J = 0.5 Hz),
4,634 (H - 1b, d, J 8.31 Hz), 4,458 (H - 1c, d, J 7.82 Hz), 2,679 (H - 3d, eq, q, J 4.63, 12.69 Hz), 2,073 (3H, s, Ac), 2,037 (3H, s, Ac), 1,731 (H-3d, ax, t, J 11.74 Hz)

Example 8 Synthesis of the compound (16)

The compound (14) (19.0 mg) was dissolved in methanol (2 ml) and 1N - $NaOCH_3$ (0.1 ml) was added thereto. The reaction mixture was stirred at room temperature for 18 hours and concentrated in vacuo. The residue was dissolved in methanol (1 ml), and tetrahydrofuran (1 ml) and $H_2O$ (one drop) were added thereto. The reaction mixture was stirred at room temperature for 19 hours. AMBERLYST® 15 (0.4 ml) was added and stirred for 10 minutes to neutralize the reaction mixture which was then filtered through Celite® and concentrated in vacuo. The residue was dissolved in 75% aqueous acetic acid (4 ml), to which 10% palladium on charcoal (20 mg) was added and hydrogen gas was introduced. The reaction mixture was stirred at 80°C for 30 minutes. After Celite filtration, the reaction mixture was concentated in vacuo. The residue was subjected to gel permeation chromatography (SEPHADEX G-25 (registered trademark), 20 ml, $H_2O$). The eluate was freeze-dried to give the compound (16) (9.1 mg, 88.3%) as crystals.

[The compound (16)]

TLC: Rf = 0.17 (n-BuOH : EtOH : $H_2O$ = 2:1:1)

$$[\alpha]_D^{25.5} - 21.4^\circ (C0.36,\ H_2O)$$

PMR (400 MHz) ($D_2O$) : 5,218 (H - 1a, d, J = 0.5 Hz), 4,623 (H -1b, d, J 8.05 Hz), 4,474 (H - 1c, d, J 7.81 Hz), 2,395 (H - 3d, eq, q, J 4.88, 12.7), 2,055 (6 H, s, Ac x 2), 1,635 (H - 3d, ax, t, J 12.70 Hz)

## Claims

**1.** Sialyloligosaccharides of the formula:

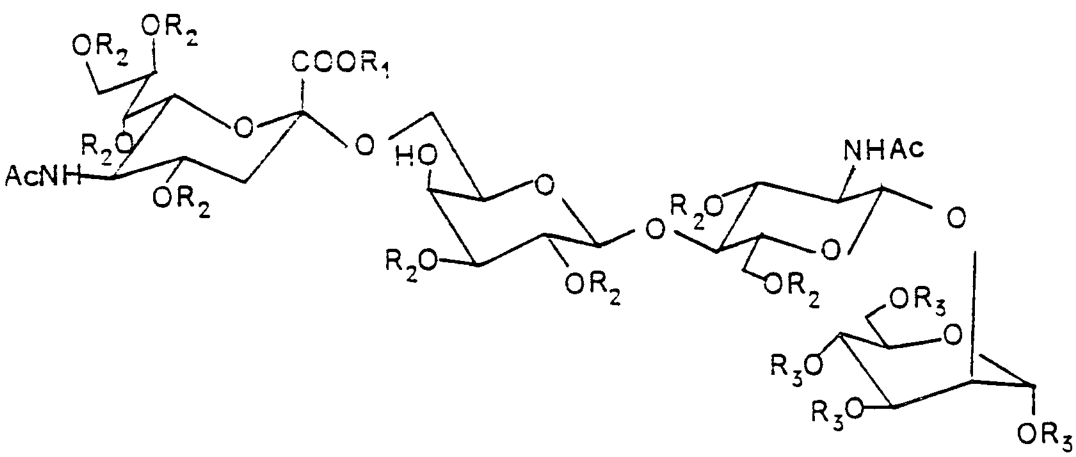

or

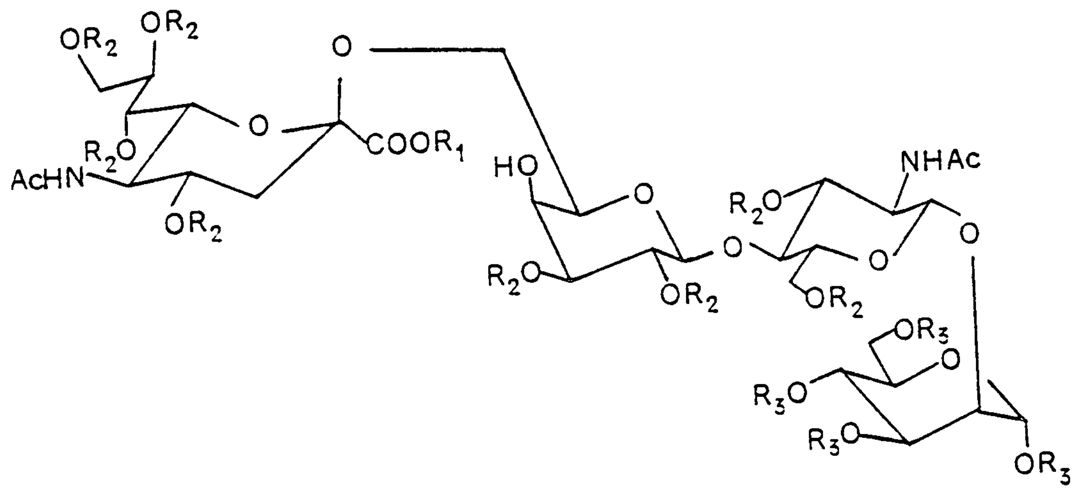

wherein $R_1$ is hydrogen or methyl, $R_2$ is hydrogen or acetyl, $R_3$ is hydrogen or benzyl, and Ac is acetyl.

**2.** The compounds of claim 1 represented by the formula:

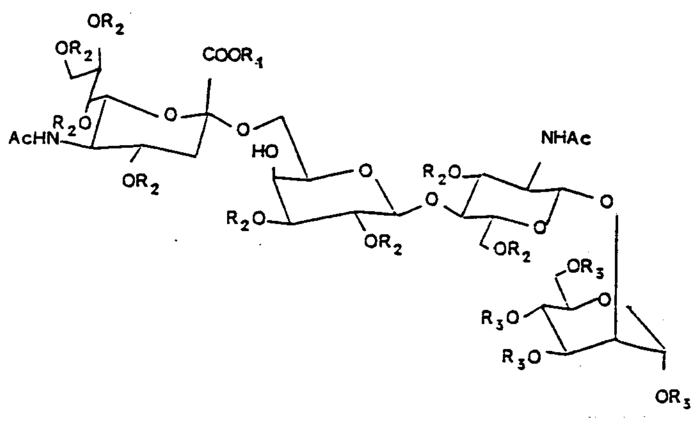

wherein $R_1$ is hydrogen or methyl, $R_2$ is hydrogen or acetyl, and $R_3$ is hydrogen or benzyl.

**3.** The compound of claim 2 wherein $R_1$ is methyl, $R_2$ is acetyl, and $R_3$ is benzyl.

**4.** The compound of claim 2 wherein $R_1$, $R_2$ and $R_3$ are hydrogen.

**5.** The compounds of claim 1 represented by the formula:

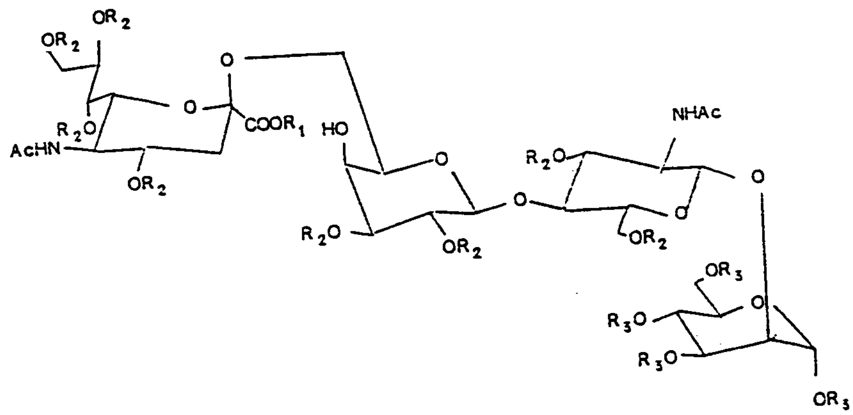

wherein $R_1$ is hydrogen or methyl, $R_2$ is hydrogen or acetyl, and $R_3$ is hydrogen or benzyl.

**6.** The compound of claim 5 wherein $R_1$ is methyl, $R_2$ is acetyl, and $R_3$ is benzyl.

**7.** The compound of claim 5 wherein $R_1$, $R_2$ and $R_3$ are hydrogen.

**8.** A method for producing sialyloligosaccharides as defined in anyone of claims 1 to 7, which comprises reacting the compound of the formula.

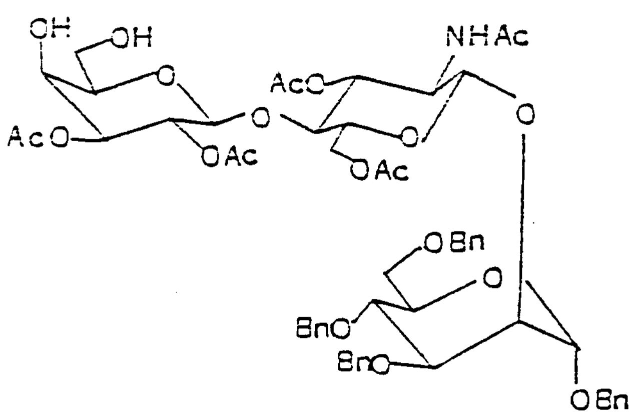

wherein Ac is acetyl and Bn is benzyl, with the compound of the formula

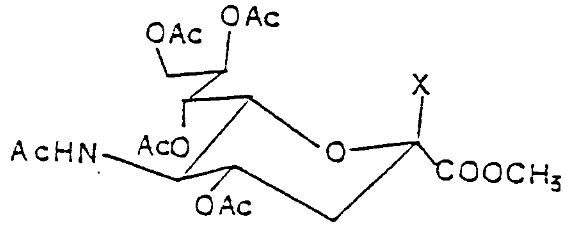

wherein X is halogen, and optionally performing deacetylation, saponification and/or debenzylation.

**9.** Compounds of the formula:

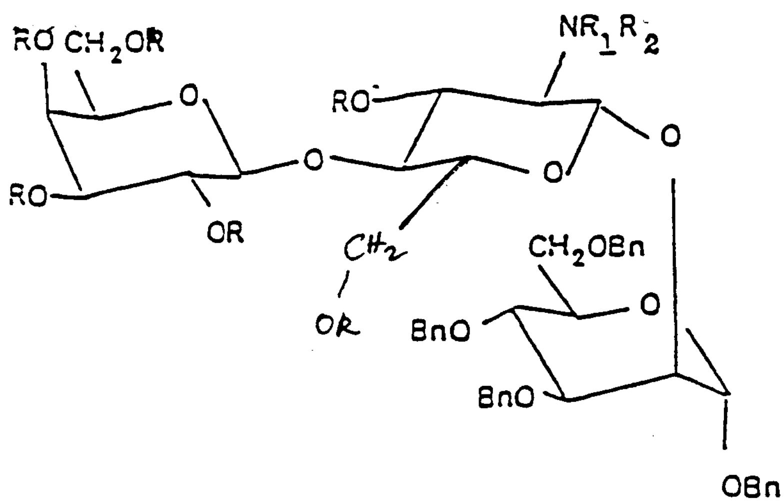

wherein R is hydrogen, $R_1$ is hydrogen, $R_2$ is acetyl, or wherein R is acetyl and $R_1$ and $R_2$ are hydrogen and Bn is benzyl.

**10.** The compound of claim 9 wherein R is acetyl, $R_1$ and $R_2$ are hydrogen.

**11.** The compound of claim 9 wherein R and $R_1$ are hydrogen, $R_2$ is acetyl.

**12.** A method for producing the compounds as defined in claim 9 which comprises reacting the compound of the formula:

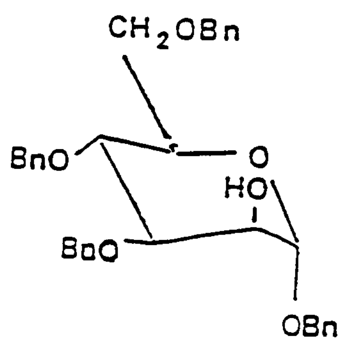

wherein Bn is benzyl, with the compound of the formula:

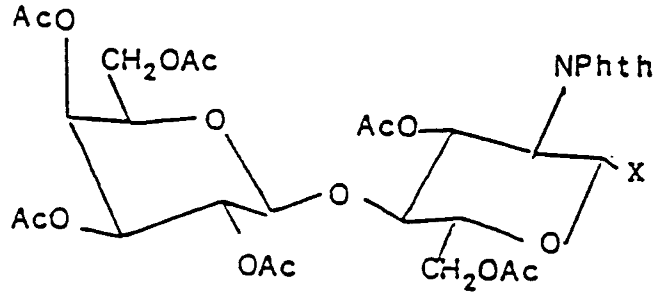

wherein X is halogen, Ac is acetyl, Phth is phthaloyl, and performing deacetylation, dephthaloylation and/or acetylation.

**13.** The compound of the formula:

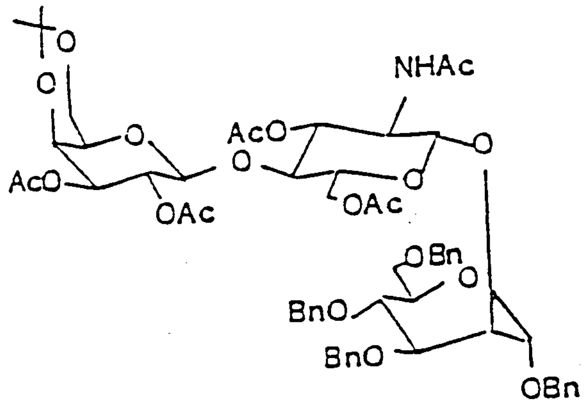

wherein Ac is acetyl and Bn is benzyl.

**14.** The compound of the formula:

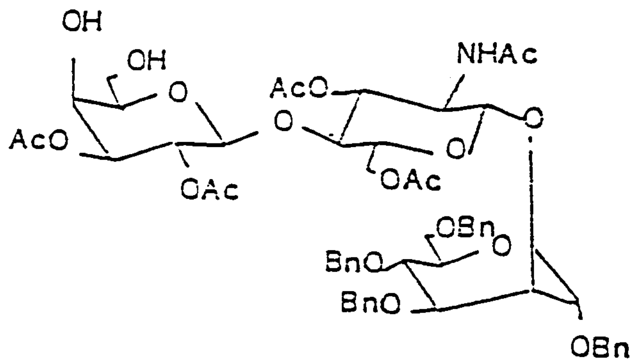

wherein Ac is acetyl and Bn is benzyl.

**15.** Pharmaceutical preparations containing sialyloligosaccharides of any of claims 1 to 7.

**Revendications**

**1.** Sialyloligosaccharides de formule :

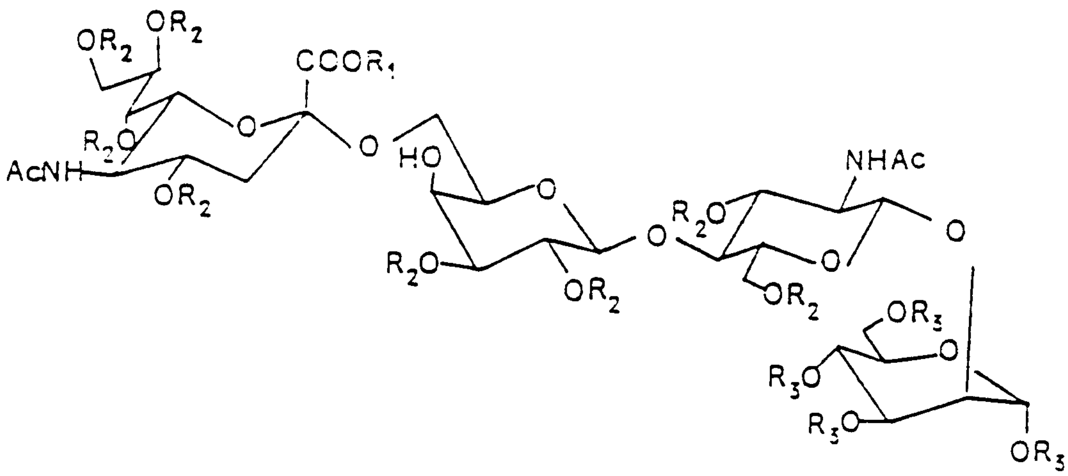

ou

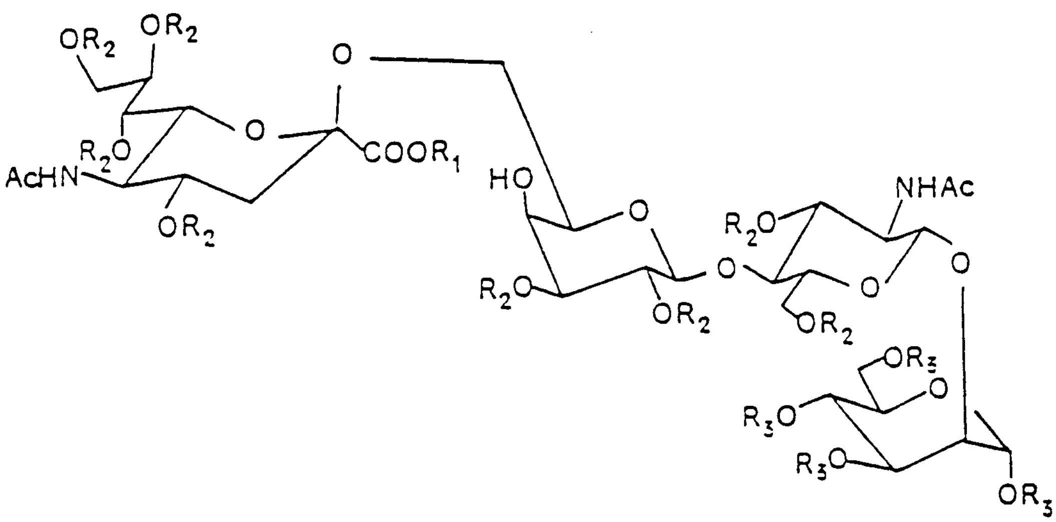

dans laquelle $R_1$ est un atome d'hydrogène ou un groupe méthyle, $R_2$ est un atome d'hydrogène ou un groupe acétyle, $R_3$ est un atome d'hydrogène ou un groupe benzyle et Ac est un groupe acétyle.

**2.** Les composés selon la revendication 1, représentés par la formule :

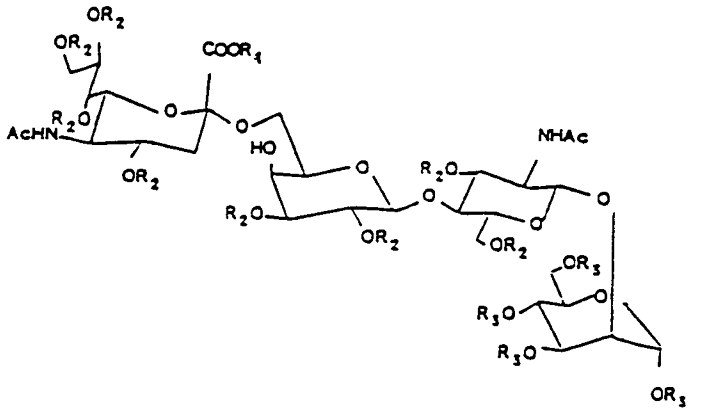

dans laquelle $R_1$ est un atome d'hydrogène ou un groupe méthyle, $R_2$ est un atome d'hydrogène ou un groupe acétyle et $R_3$ est un atome d'hydrogène ou un groupe benzyle.

3. Le composé selon la revendication 2, selon lequel $R_1$ est un groupe méthyle, $R_2$ est un groupe acétyle et $R_3$ est un groupe benzyle.

4. Le composé selon la revendication 2, selon lequel $R_1$, $R_2$ et $R_3$ sont des atomes d'hydrogène.

5. Les composés selon la revendication 1, représentés par la formule :

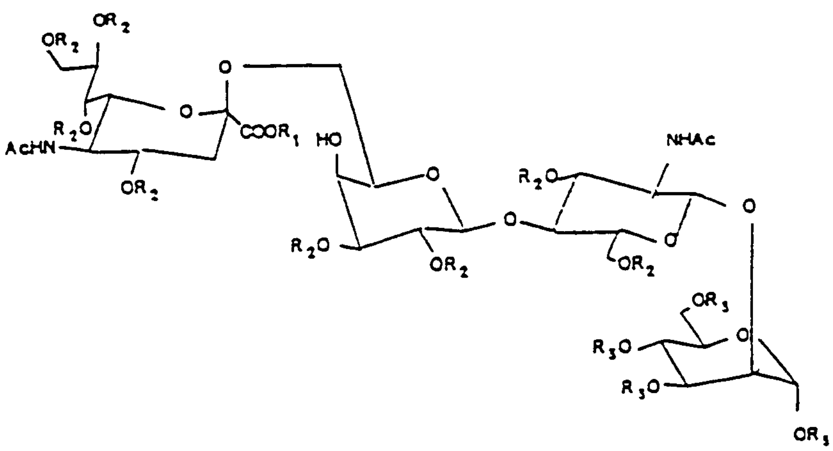

dans laquelle $R_1$ est un atome d'hydrogène ou un groupe méthyle, $R_2$ est un atome d'hydrogène ou un groupe acétyle, et $R_3$ est un atome d'hydrogène ou un groupe benzyle.

6. Le composé selon la revendication 5, selon lequel $R_1$ est un groupe méthyle, $R_2$ est un groupe acétyle et $R_3$ est un groupe benzyle.

7. Le composé selon la revendication 5, selon lequel $R_1$, $R_2$ et $R_3$ sont des atomes d'hydrogène.

8. Une méthode de production des sialyloligosaccharides tels que définis dans l'une quelconque des revendications 1 à 7, qui comprend la réaction du composé de formule :

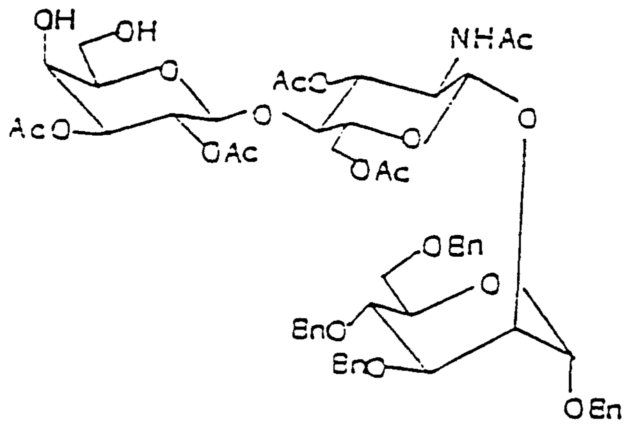

dans laquelle Ac est un groupe acétyle et Bn est un groupe benzyle avec le composé de formule :

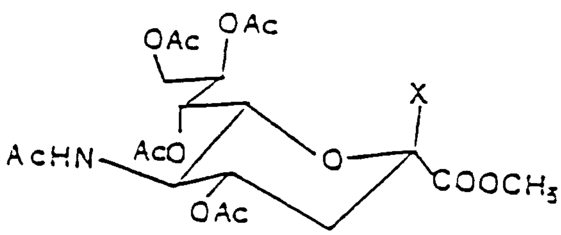

dans laquelle X est un atome d'halogène, puis on procède éventuellement à la désacétylation, à la saponification et/ou à la débenzylation.

**9.** Composés de formule :

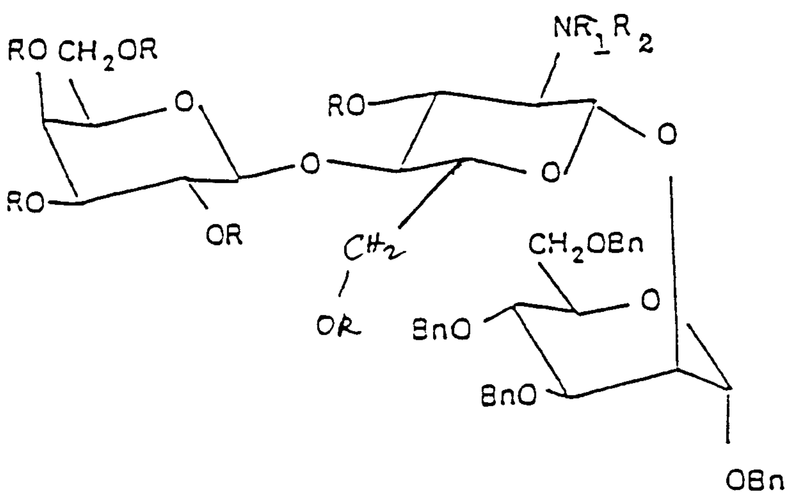

dans laquelle R est un atome d'hydrogène, $R_1$ est un atome d'hydrogène, $R_2$ est un groupe acétyle, ou dans laquelle R est un groupe acétyle et $R_1$ et $R_2$ sont des atome d'hydrogène et Bn est un groupe benzyle.

**10.** Le composé selon la revendication 9, dans lequel R est un groupe acétyle, $R_1$ et $R_2$ sont des atomes d'hydrogène.

**11.** Le composé selon la revendication 9, selon lequel R et $R_1$ sont des atomes d'hydrogène, et $R_2$ est un

groupe acétyle.

**12.** Une méthode de production du composé tel que défini dans la revendication 9 qui comprend la réaction du composé de formule :

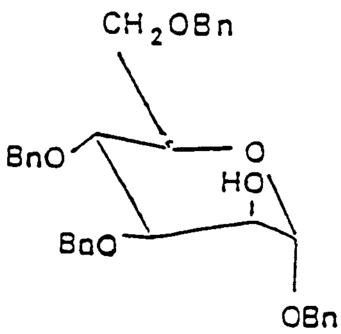

dans laquelle Bn est un groupe benzyle avec le composé de formule :

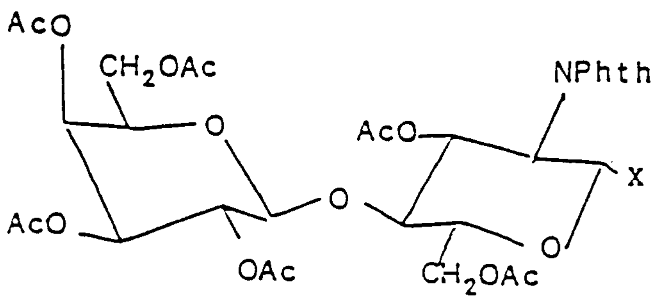

dans laquelle X est un atome d'halogène, Ac est un groupe acétyle, Phth est un groupe phtaloyle et on conduit la désacétylation, la déphtaloylation et/ou l'acétylation.

**13.** Le composé de formule :

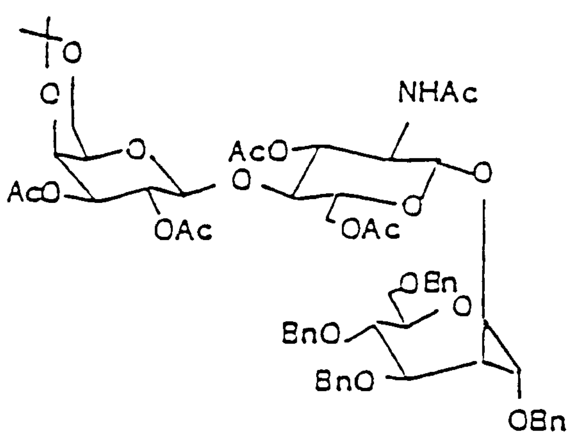

dans laquelle Ac est un groupe acétyle et Bn est un groupe benzyle.

**14.** Le composé de formule :

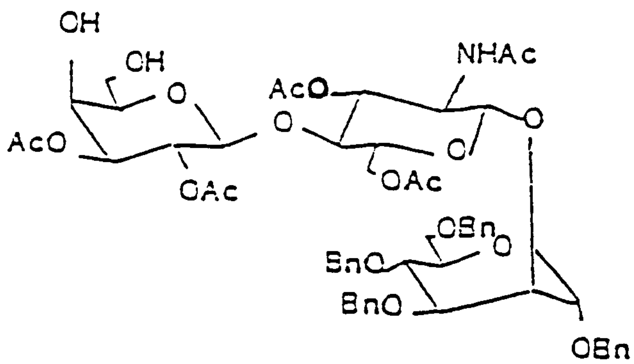

dans laquelle Ac est un groupe acétyle et Bn est un groupe benzyle.

**15.** Préparations pharmaceutiques contenant les sialyloligosaccharides selon l'une quelconque des revendications 1 à 7.

## Patentansprüche

**1.** Sialyloligosaccharide der Formel

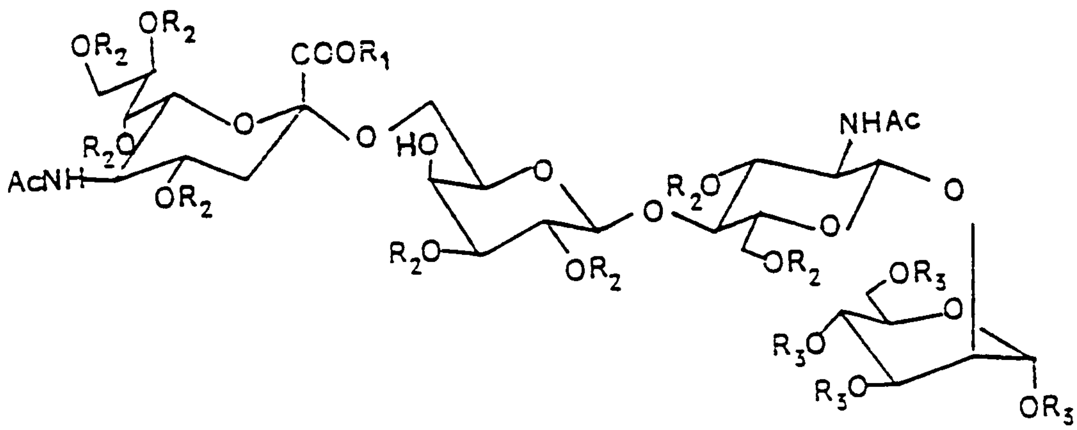

oder

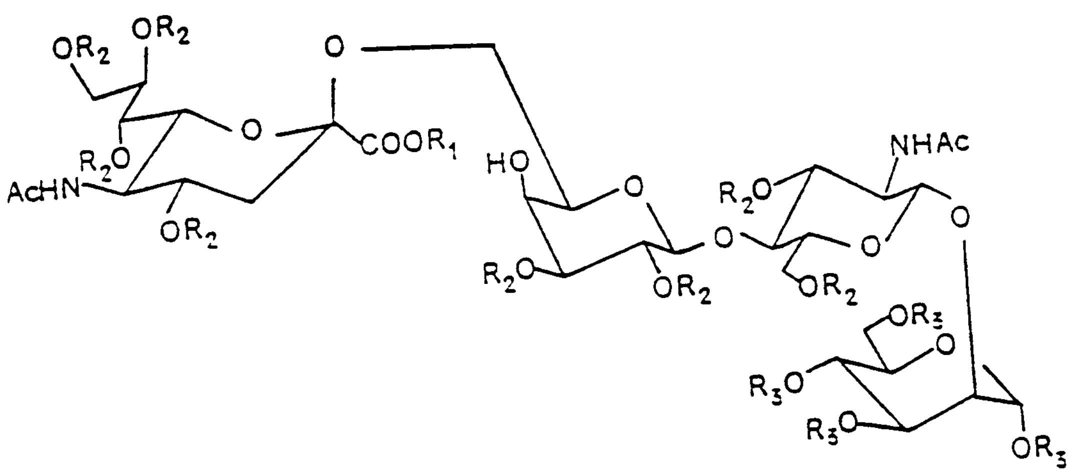

in denen $R_1$ ein Wasserstoffatom oder eine Methylgruppe ist, $R_2$ ein Wasserstoffatom oder eine

Acetylgruppe ist, $R_3$ ein Wasserstoffatom oder eine Benzylgruppe ist und Ac eine Acetylgruppe ist.

2. Verbindungen nach Anspruch 1 der Formel:

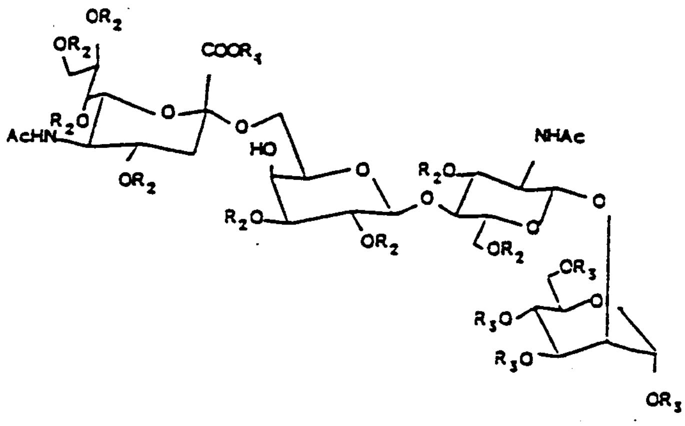

in der $R_1$ ein Wasserstoffatom oder eine Methylgruppe ist, $R_2$ ein Wasserstoffatom oder eine Acetylgruppe ist und $R_3$ ein Wasserstoffatom oder eine Benzylgruppe ist.

3. Verbindung nach Anspruch 2, in der $R_1$ eine Methylgruppe ist, $R_2$ eine Acetylgruppe ist und $R_3$ eine Benzylgruppe ist.

4. Verbindung nach Anspruch 2, in der $R_1$, $R_2$ und $R_3$ Wasserstoffatome sind.

5. Verbindungen nach Anspruch 1 der Formel:

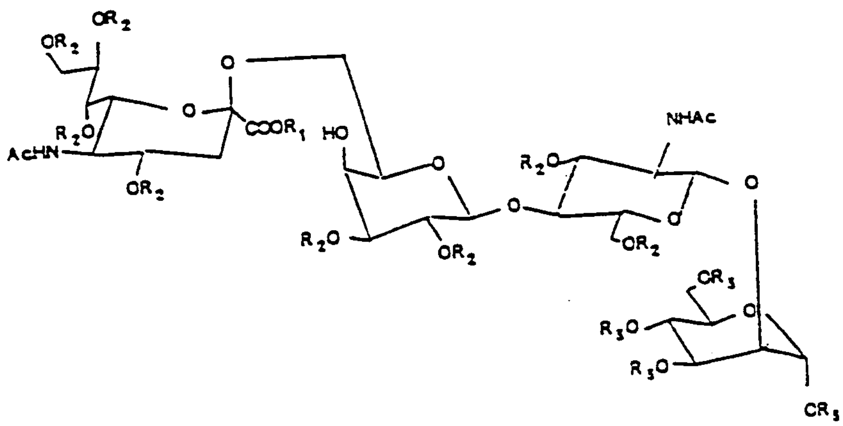

in der $R_1$ ein Wasserstoffatom oder eine Methylgruppe ist, $R_2$ ein Wasserstoffatom oder eine Acetylgruppe ist und $R_3$ ein Wasserstoffatom oder eine Benzylgruppe ist.

6. Verbindung nach Anspruch 5, in der $R_1$ eine Methylgruppe ist, $R_2$ eine Acetylgruppe ist und $R_3$ eine Benzylgruppe ist.

7. Verbindung nach Anspruch 5, in der $R_1$, $R_2$ und $R_3$ Wasserstoffatome sind.

8. Verfahren zur Herstellung von Sialyloligosacchariden nach einem der Ansprüche 1 bis 7, das die Umsetzung der Verbindung der Formel

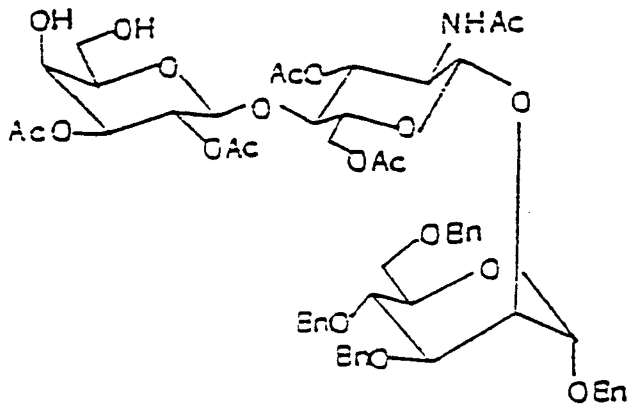

in der Ac eine Acetylgruppe und Bn eine Benzylgruppe ist, mit der Verbindung der Formel

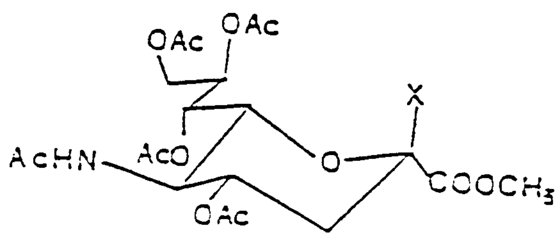

in der X ein Halogenatom ist, und gegebenenfalls die Durchführung einer Entacetylierung, Verseifung und/oder Entbenzylierung, umfaßt.

**9.** Verbindungen der Formel:

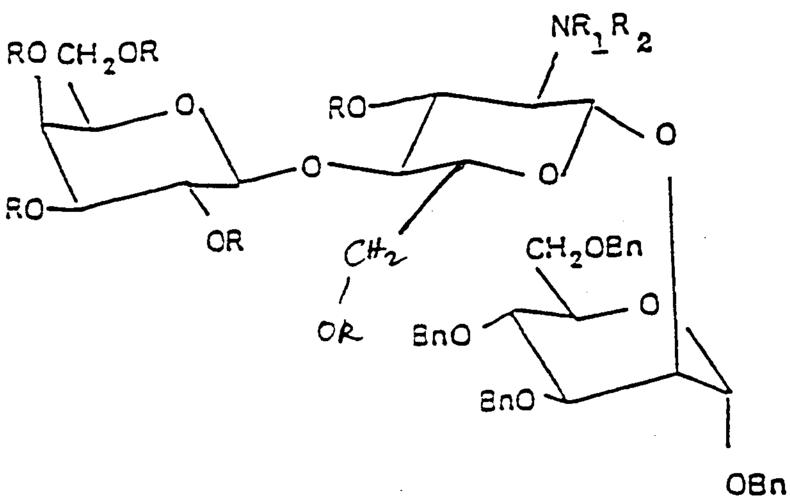

in der R ein Wasserstoffatom ist, $R_1$ ein Wasserstoffatom ist, $R_2$ eine Acetylgruppe ist, oder in der R eine Acetylgruppe ist, $R_1$ und $R_2$ Wasserstoffatome sind und Bn eine Benzylgruppe ist.

**10.** Verbindung nach Anspruch 9, in der R eine Acetylgruppe ist und $R_1$ und $R_2$ Wasserstoffatome sind.

**11.** Verbindung nach Anspruch 9, in der R und $R_1$ Wasserstoffatome sind und $R_2$ eine Acetylgruppe ist.

**12.** Verfahren zur Herstellung der Verbindungen nach Anspruch 9, das die Umsetzung der Verbindung der Formel

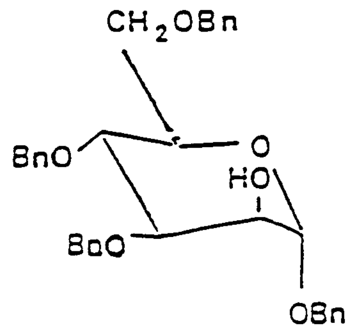

in der Bn eine Benzylgruppe ist, mit der Verbindung der Formel

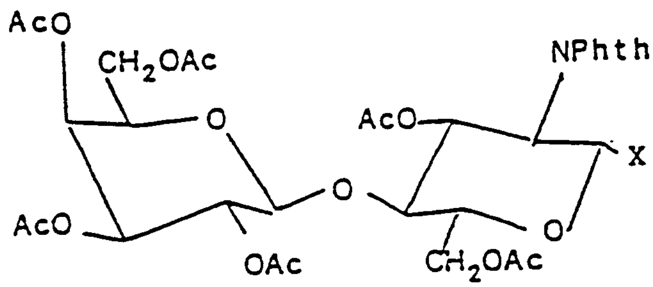

in der X ein Halogenatom ist, Ac eine Acetylgruppe ist, Phth eine Phthaloylgruppe ist, und die Durchführung einer Entacetylierung, die Entfernung der Phthaloylgruppe und/oder eine Acetylierung umfaßt.

**13.** Verbindung der Formel

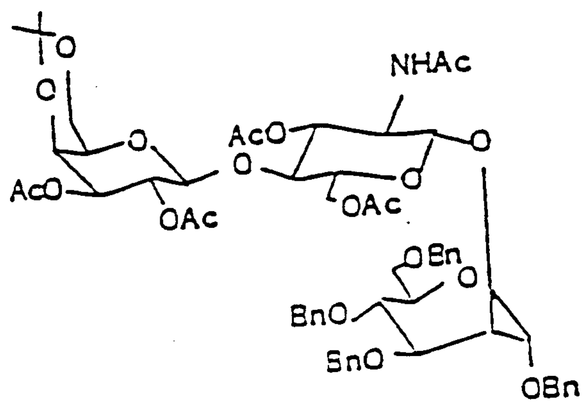

in der Ac eine Acetylgruppe ist und Bn eine Benzylgruppe ist.

**14.** Verbindung der Formel

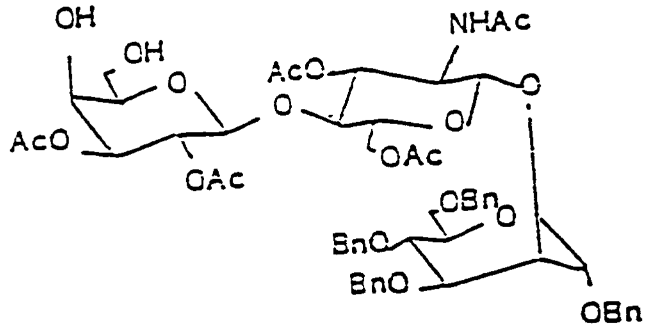

in der Ac eine Acetylgruppe ist und Bn eine Benzylgruppe ist.

**15.** Arzneimittel, enthaltend Sialyloligosaccharide nach einem der Ansprüche 1 bis 7.